# EUROPÄISCHE PATENTANMELDUNG

(11) **EP 1 371 978 A1**
(43) Veröffentlichungstag der Anmeldung: **17.12.2003**
(21) Anmeldenummer: 02013000.1
(22) Anmeldetag: 12.06.2002
(51) Int. Cl.: G01N 33/36, G01N 22/00, G01N 22/04

(54) **Mikrowellenvorrichtung zur Qualitätsprüfung strangförmiger Materialien**

(71) Anmelder: TEWS ELEKTRONIK Dipl.-Ing. Manfred Tews, 22459 Hamburg (DE)
(72) Erfinder: Herrmann,Rainer,Dipl.-Phys., 20253 Hamburg (DE); Tews,Manfred,Dipl.-Ing., 22459 Hamburg (DE); Schlemm,Udo,Dipl.-Phys., 20357 Hamburg (DE)
(74) Vertreter: Glawe, Delfs, Moll & Partner

(57) **Zusammenfassung**

Die Vorrichtung zur Qualitätsprüfung von strangförmigen Materialien (2) in Form von Garnen, Vorgarnen, Faserbändern und dergleichen zeichnet sich dadurch aus, daß die Meßeinrichtung auf der Basis der Mikrowellentechnik einen Mikrowellenresonator (3) und Einrichtungen (4) zum Bestimmen der Verschiebung der Resonanzfrequenz und der Verbreiterung der Resonanzkurve durch das strangförmige Material aufweist.

## Beschreibung

Die Erfindung betrifft eine Vorrichtung zur Qualitätsprüfung von strangförmigen Materialien in Form von Garnen, Vorgarnen, Faserbändern und dergleichen mit einer Meßeinrichtung, einer Auswertungsschaltung und mechanischen Einrichtungen zum Transport der strangförmigen Materialien durch die Meßeinrichtung.

Für die Qualitätsprüfung von Garnen, Vorgarnen und Faserbändern ist eine Detektion von Qualitätsschwankungen, insbesondere von Masseschwankungen, d. h. eine Überprüfung ihrer Homogenität notwendig. Es müssen dann Fehler bezüglich Masseänderung erfaßt werden. Anhand der Häufigkeit und Intensität der Fehler kann eine Qualitätsbewertung des Meßgutes erfolgen. Diese Qualitätsbewertung soll vorzugsweise, aber nicht ausschließlich mit einem Labormeßplatz bewirkt werden. Die Messung soll unabhängig von Parametern wie Materialfeuchte, Materialfarbe, Materialsorte (z. B. Anbaugebiet von Baumwolle) erfolgen.

Es sind verschiedene Vorrichtungen zur Qualitätsprüfung von strangförmigen Materialien bekannt, die allerdings Nachteile aufweisen. Die Dicke von insbesondere Faserbändern kann mechanisch gemessen werden, indem das Faserband durch ein Paar von Walzen hindurchgeführt wird, von denen wenigstens eine beweglich ist (DE 298 23 928 U1). Durch ein Tastelement kann dann die durch Dickenänderung bewirkte Auslenkung einer der Walzen bestimmt werden. Dieses Verfahren mag für Faserbänder einer konstanten Breite nutzbar sein. Für verhältnismäßig dünne Garne ist es nicht geeignet, da Dickenänderungen in Axialrichtung nur dann erfaßt werden können, wenn wie beim Stand der Technik die Walzen als Nut- und Federwalzen ausgebildet sind.

Es ist auch bekannt, Messungen der Masse mit einer aus Lichtsender und Lichtempfänger bestehenden Anordnung vorzunehmen (DE 29 12 558 C2). Hiermit können aber nur optische Eigenschaften infolge oberflächlicher Reflektionen gemessen werden, die dann die Masse bzw. Masse pro Länge des Materials nicht wiedergeben. Verfälschungen können z. B. bei Schwankungen der Farbe des zu messenden Strangs auftreten. Dieses Verfahren des Standes der Technik ist auch in erster Linie nur für Faserbänder vorgesehen.

Dies gilt auch für ein Verfahren, bei dem Schall durch das Material gesendet wird und aus der Änderung der Dämpfung auf Änderungen der Materialeigenschaften geschlossen wird (DE 32 37 357 C2). Eine eindeutige Bestimmung der Masse ist dadurch nicht möglich, da die Dämpfung auch durch andere Eigenschaften, z. B. die Feuchte des Materials beeinflußt werden kann. Außerdem ist dieses Material für dünnere Garne kaum zu verwenden, da nicht der gesamte Schall durch das Garn hindurchgelenkt werden kann.

Schließlich ist es bekannt, das strangförmige Material durch zwei Platten eines Kondensators hindurchlaufen zu lassen (DE-OS 20 41 044). Solche kapazitiven Meßverfahren haben den Nachteil, daß die Signale sehr empfindlich auf Änderungen der Feuchte wegen der hohen Dielektrizitätskonstante von Wasser reagieren. Es kann dadurch der Einfluß von Feuchtevariationen nicht kompensiert werden. Das Material muß daher vor der kapazitiven Messung auf einen genau definierten und bekannten Feuchtewert stabilisiert werden, was ohne weiteres ein bis zwei Tage auch bei gleichmäßigen Temperaturen und sonstigen konstanten Bedingungen erfordern kann. Eine schnelle Messung der Qualität ist damit nicht möglich. Die Feuchtefluktuationen sind dabei besonders störend, je produktionsnaher das Textilmaterial in der Teststation untersucht wird, wenn also nicht für eine genügende Homogenisierung des Materials gesorgt werden kann. Ein präzises Abbild der Qualität des aktuellen Produkts, das frei von Feuchtefluktuationen ist, ergibt sich erst nach einer entsprechend langen Lagerdauer unter entsprechenden Bedingungen. Aber auch dann setzen Feuchte-Inhomogenitäten aufgrund variabler Porenstrukturen, wie sie in allen Produkten auftreten, der Meßpräzision eine deutliche Grenze.

Die Aufgabe der Erfindung besteht in der Schaffung einer Vorrichtung der eingangs genannten Art, mit der schnell und zuverlässig Qualitätsmessungen ohne störenden Einfluß von Feuchteschwankungen durchgeführt werden können.

Die erfindungsgemäße Lösung besteht darin, daß die Meßeinrichtung auf der Grundlage der Mikrowellentechnik einen Mikrowellenresonator und Einrichtungen zum Bestimmen der Verschiebung der Resonanzfrequenz und der Verbreiterung der Resonanzkurve durch das strangförmige Material aufweist.

"Bestimmen der Verschiebung der Resonanzfrequenz und der Verbreiterung der Resonanzkurve" bedeutet dabei nicht, daß diese Bestimmung tatsächlich und direkt durch schrittweises Abfahren der Resonanzkurve vorgenommen wird. Diese Bestimmung kann auch indirekt durch Messung an einigen wenigen Punkten der Resonanzkurve vorgenommen werden, wobei dann gewisse Annahmen über die Form dieser Resonanzkurve gemacht werden.

Die Messung wird also nicht mechanisch, optisch, akustisch oder kapazitiv vorgenommen, sondern mit Mikrowellen. Mit der Mikrowellentechnik steht ein genaues Verfahren zur Verfügung, um Qualitätsparameter, insbesondere die Masse unabhängig von der Feuchtigkeit zu bestimmen (WO 91/12518). Die Mikrowellenmeßtechnik erlaubt es dabei auch, mit sehr hohen Geschwindigkeiten des strangförmigen Materials zu arbeiten, da keine mechanisch bewegten Teile vorgesehen sind, die auf Dickeänderungen reagieren müssen. Vielmehr erfolgt die gesamte Erfassung und Auswertung elektronisch.

Die Messungen können mit Frequenzen von 300 MHz bis 30 GHz durchgeführt werden. Insbesondere haben sich aber Frequenzen zwischen 1 GHz bis 15 GHz als besonders geeignet erwiesen.

Um genaue Messungen zu ermöglichen, sollte durch das durchlaufende strangförmige Material das Feld des Resonators möglichst stark beeinflußt werden. Das Feld des Resonators sollte also weitgehend auf den Raumbereich konzentriert sein, den das strangförmige Material durchläuft. Während man für breite Faserbänder verhältnismäßig große Resonatoren benötigt, durch die das Material hindurchlaufen muß, wird man für dünnere Garne oder Vorgarne kleinere Resonatoren verwenden. Die unterschiedlichen Resonatoren werden dabei über Umschalter mit der Auswertungsschaltung verbunden, so daß immer derjenige Resonator mit der Auswertungsschaltung verbunden ist, durch den gerade das zu messende Material hindurchläuft. Andererseits oder zusätzlich können die Mikrowellenresonatoren verschiebbare Elemente zum Verändern der Mikrowellenfeldkonfiguration aufweisen, damit auch in einem größeren Resonator, durch den ein Material mit geringeren Ausmessungen hindurchläuft, die Mikrowellenstärke auf das Material konzentriert werden kann.

Die Vorrichtung ist zweckmäßigerweise als Labormeßplatz ausgebildet, damit die strangförmigen Materialien vor der Verarbeitung überprüft werden können.

Zusätzlich zu den bereits erwähnten Vorteilen, insbesondere gegenüber dem kapazitiven Verfahren, hat die Mikrowellentechnik den Vorteil, daß die Vorrichtung unmittelbar nach dem Einschalten in Betrieb genommen werden kann. Die Mikrowellengeräte benötigen nicht wie bei den empfindlichen kapazitiven Verfahren eine Warmlaufphase.

Mit der erfindungsgemäßen Vorrichtung kann nicht nur die Feuchte masseunabhängig gemessen werden. Es ist vielmehr insbesondere eine genaue Massemessung möglich, bei der der Einfluß der Feuchte automatisch kompensiert wird, also feuchteunabhängige Massemessungen möglich sind. Letzteres ist von besonderer Bedeutung, da eine Variation der Materialfeuchtigkeit sich durch die große Dielektrizitätskonstante des Wassers empfindlich als Störung einer exakten Massemessung erweist. Durch die automatische Kompensation des Mikroweilen-Massemeßwertes mit Hilfe des Mikrowellen-Feuchtemeßwertes kann diese Fehlerquelle ausgeschieden werden. Die mit Hilfe der Mikrowellenmethode gemessene Masse entspricht so der tatsächlichen Masse des Produkts. Führt man zusätzlich eine Feuchtekalibration auf Basis eines der gängigen Referenzverfahrens durch (die innerhalb eines Materialtyps weitgehend sortenunabhängig ist), so ermöglicht das Mikrowellenverfahren auch die Messung der Trockenmaterialmasse oder die Masse bei einer frei definierbaren Zielfeuchte.

Wenn hier von "Masse" die Rede ist, so ist damit immer die Masse pro Längeneinheit gemeint.

Durch die Erfindung wird also eine Vorrichtung geschaffen, mit der die Qualität, insbesondere die Masse pro Länge von strangförmigen Materialien in Form von Garnen, Vorgarnen, Faserbändern und dergleichen genau bestimmt werden kann. Insbesondere ist dabei die Vorrichtung als Laborplatz ausgebildet. Die Vorrichtung ist dabei zur Auswertung der Daten vorzugsweise so ausgebildet, daß sie an einen PC angeschlossen werden kann.

Die Erfindung wird im folgenden anhand von vorteilhaften Ausführungsformen unter Bezugnahme auf die beigefügten Zeichnungen beschrieben. Es zeigen in schematischer Darstellung:
- Fig. 1: den prinzipiellen Aufbau der erfindungsgemäßen Vorrichtung;
- Fig. 2: einen für die Vorrichtung geeigneten Mikrowellenresonator;
- Fig. 3: einen anderen für die Vorrichtung geeigneten Mikrowellenresonator;
- Fig. 4: eine Anordnung zur Messung von verschiedenen Größen von strangförmigen Materialien;
- Fig. 5: eine andere Anordnung zur Messung von verschiedene Größen strangförmiger Materialien; und
- Fig. 6: einen Mikrowellenresonator, der für die Messung von Garnen mit geringem Durchmesser geeignet ist.

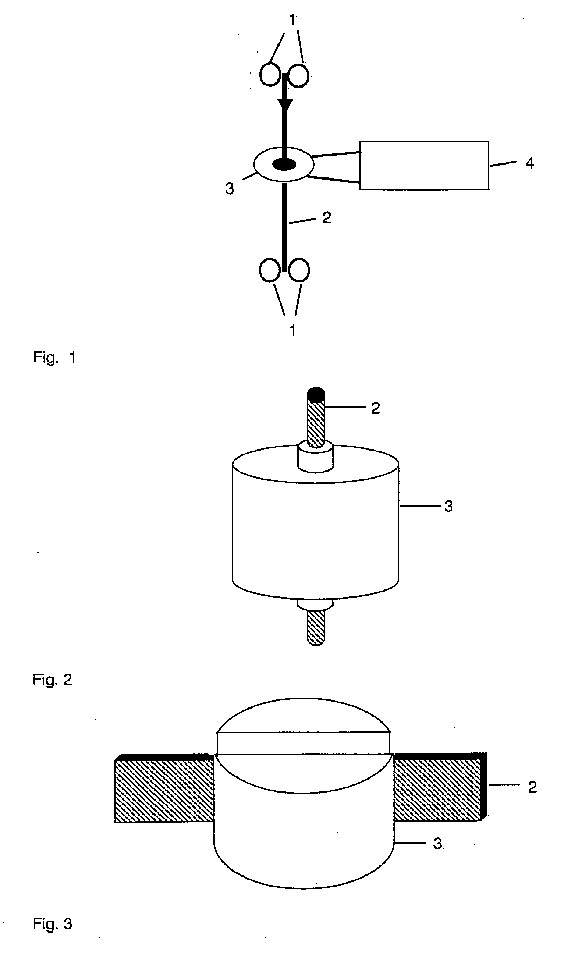

Fig. 1 zeigt den prinzipiellen Aufbau der erfindungsgemäßen Vorrichtung. Über Strangführungen, die bei 1 dargestellt sind, wird das strangförmige Material 2 durch einen Mikrowellenresonator 3 geführt, der mit einer Meßelektronik 4 verbunden ist. Durch die Meßelektronik werden Mikrowellen in den Hohlraum 3 eingestrahlt. Die aus dem Hohlraum ausgekoppelte Mikrowellenstrahlung wird detektiert. Es wird dabei sowohl die Verschiebung der Resonanzfrequenz als auch die Verbreiterung der Resonanzkurve durch das hindurchgeführte strangförmige Material 2 bestimmt.

Fig. 2 zeigt einen Resonator 3, der bei verschiedenen Arbeitsfrequenzen für strangförmiges Material 2 unterschiedlicher Dicke geeignet ist. Bei niedrigen Frequenzen bis ca. 5 GHz ist er zur Messung von dicken Faserbändern geeignet, bei hohen Frequenzen zur Messung dünner Garne. Fig. 3 zeigt einen Resonator 3, der für strangförmiges Material, das keinen kreisförmigen, sondern einen länglichen Querschnitt hat, geeignet ist.

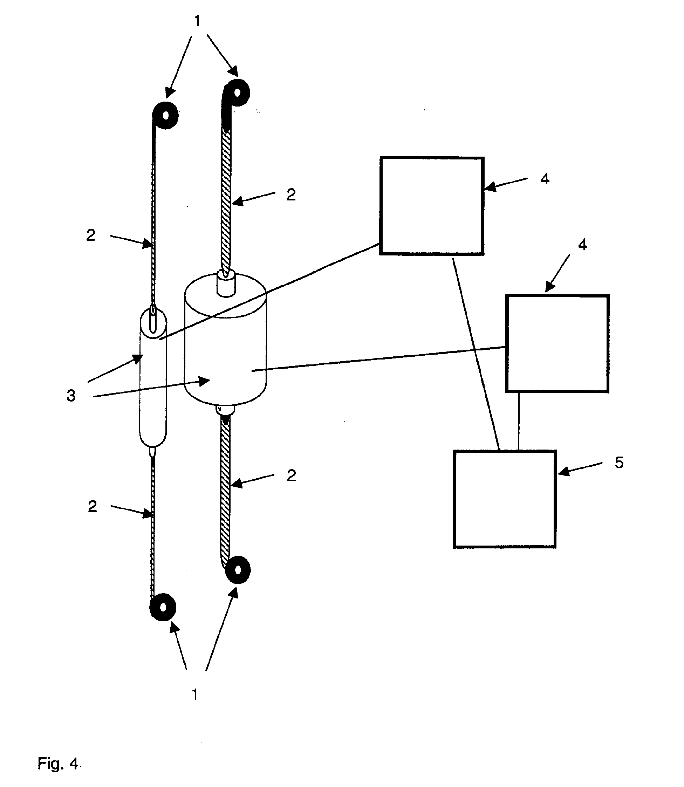

Bei der Ausführungsform der Fig. 4 sind gleichzeitig mehrere Resonatoren 3 vorgesehen, die für dünnes Material (links) und dickes Material (rechts) vorgesehen sind. Jeder dieser Mikrowellenresonatoren ist mit einer eigenen Mikrowellenelektronik verbunden, wobei der kleinere Resonator mit höheren Mikrowellenfrequenzen betrieben wird als der größere Resonator. Die Bauform der Resonatoren kann in diesem Falle für alle Resonatoren die gleiche sein. Die Größen der Resonatoren hängen aber von der Arbeitsfrequenz ab. Im höheren Frequenzbereich werden kleinere Resonatoren betrieben, die in der Lage sind, Meßgut mit sehr geringer Maße zu messen. Für dickere Faserbänder dagegen kommen größere Resonatoren bei niedrigerer Frequenz zum Einsatz. Als mögliche Resonatoren werden in diesem Falle zylindrische Resonatoren eingesetzt, die vom Meßgut im Bereich der Zylinderachse durchdrungen werden. Beispielsweise werden die kreiszylindrischen Resonatoren im E₀₁₀-Mode betrieben. Der Durchmesser der Resonatoren ist abhängig von der Wellenlänge.

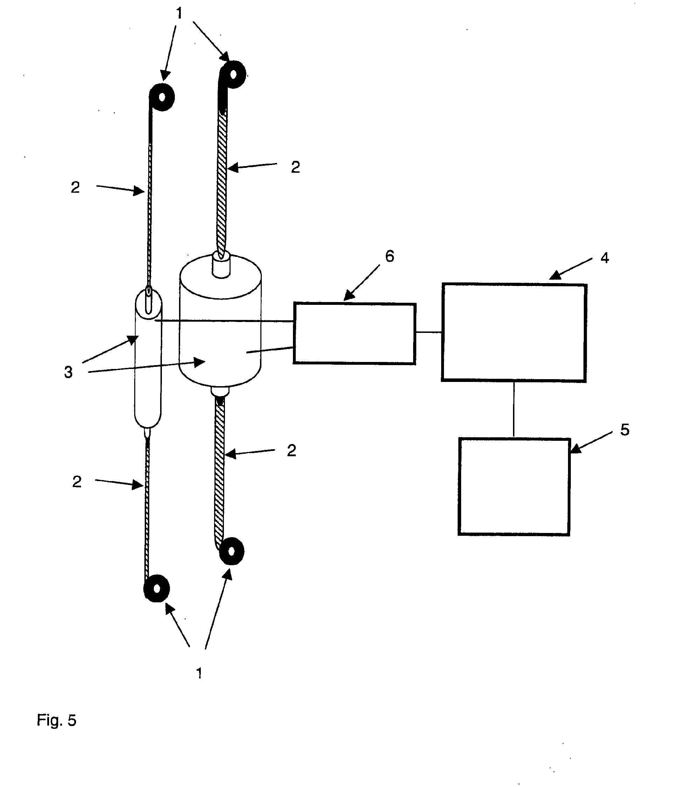

Bei der Ausführungsform der Fig. 5 ist vorteilhafterweise nur eine Mikrowellenelektronik 4 vorgesehen, die jeweils mit einem Umschalter 6 mit dem Mikrowellenresonator 3 verbunden wird, durch den gerade strangförmiges Material 2 hindurchgeführt wird. In diesem Falle ist nur eine Mikrowellen-Elektronik in Kombination mit einem Umschalter nötig, der die verschiedenen Resonatoren ansteuert. Bei Messungen in niedrigem Bereich von 2 bis 3 GHz können für die Messung massereicherer Bänder zylindrischere Resonatoren verwendet werden. Für sehr dünne Garne muß trotz der relativ großen Wellenlänge das Feld im Resonator auf kleinstem Raum konzentriert werden, um eine genügende Empfindlichkeit der Resonatoren zu gewährleisten.

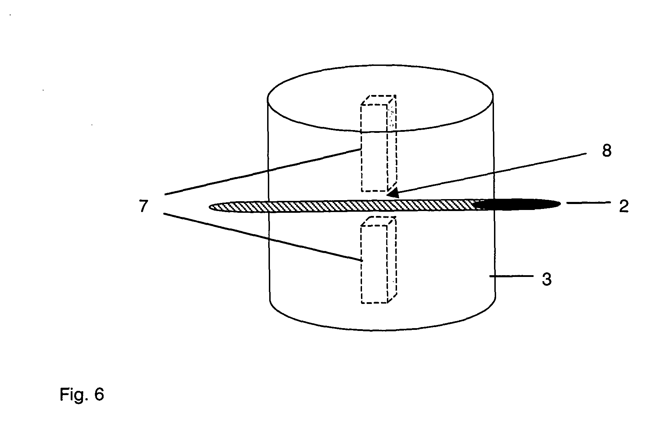

Um eine genaue Messung zu ermöglichen, sollte das Mikrowellenfeld des Resonators 3 nicht nur für den Fall dünner Garne im wesentlichen auf das strangförmige Material 2 konzentriert werden. Dies geschieht bei der Ausführungsform der Figur 4 durch verschieden große Resonatoren mit sehr unterschiedlichen Resonanzfrequenzen. Bei der Ausführungsform der Figur 5 werden alle Bandmassen in demselben Frequenzbereich gemessen. Dies geschieht durch Feldkonzentration mit Hilfe von interner Strukturen der Resonatoren. Bei der Ausführungsform der Figur 6 sind metallische Stempel 7 vorgesehen, mit denen das Mikrowellenfeld so verändert werden kann, daß es bei 8 konzentriert ist, so daß eine genaue Messung des strangförmigen Materials 2 möglich ist.

## Patentansprüche

1. Vorrichtung zur Qualitätsprüfung von strangförmigen Materialien (2) in Form von Garnen, Vorgarnen, Faserbändern und dergleichen mit einer Meßeinrichtung, einer Auswertungsschaltung (4) und mechanischen Einrichtungen (1) zum Transport der strangförmigen Materialien (2) durch die Meßeinrichtung, **dadurch gekennzeichnet, daß** die Meßeinrichtung auf der Grundlage der Mikrowellentechnik einen Mikrowellenresonator (3) und Einrichtungen (4) zum Bestimmen der Verschiebung der Resonanzfrequenz und der Verbreiterung der Resonanzkurve durch das strangförmige Material (2) aufweist.

2. Vorrichtung nach Anspruch 1, **dadurch gekennzeichnet, daß** sie zum Messen der Masse pro Länge der strangförmigen Materialien (2) ausgebildet ist.

3. Vorrichtung nach Anspruch 1, **dadurch gekennzeichnet, daß** sie zum Messen des Feuchtegehaltes der strangförmigen Materialien (2) ausgebildet ist.

4. Vorrichtung nach einem der Ansprüche 1 bis 3, **dadurch gekennzeichnet, daß** sie mit Frequenzen von 300 MHz bis 30 GHz, insbesondere 1 GHz bis 15 GHz betreibbar ist.

5. Vorrichtung nach einem der Ansprüche 1 bis 4, **dadurch gekennzeichnet, daß** sie mehrere Resonatoren (3) aufweist, die über Umschalter (6) mit der Auswertungsschaltung (4) verbindbar sind.

6. Vorrichtung nach einem der Ansprüche 1 bis 5, **dadurch gekennzeichnet, daß** die Mikrowellenresonatoren (3) Elemente (7) zur Konzentration des Mikrowellenfeldes aufweisen.

7. Vorrichtung nach einem der Ansprüche 1 bis 6, **dadurch gekennzeichnet, daß** sie als Labormeßplatz ausgebildet ist.
